# EUROPEAN PATENT SPECIFICATION

(11) **EP 1 574 198 B1**
(45) Date of publication and mention of the grant of the patent: **25.07.2007**
(21) Application number: 04425162.7
(22) Date of filing: 10.03.2004
(51) Int. Cl.: A61H 23/00

(54) **Instrument for the medical treatment of biological tissues by shock-wave therapy**
Instrument zur medizinischen Behandlung von Gewebe mittels Schockwellen
Instrument pour le traitement médical de tissue par ondes de choc

(43) Date of publication of application: 14.09.2005
(73) Proprietor: Elettronica Pagani S.r.l., 20037 Paderno Dugnano (MI) (IT)
(72) Inventor: Grassi, Gianluca, 20037 Paderno Dugnano MI (IT); Marelli, Mirco, 20037 Paderno Dugnano MI (IT)
(74) Representative: Arena, Giovanni

(56) References cited:
- WO-A-01/00094
- DE-A- 10 215 416
- US-A- 5 727 556
- US-B1- 6 413 230

## Description

The present invention relates to an instrument for the medical treatment of biological tissues by shock-wave therapy.

For the treatment of various biological tissue pathologies such as for example tendonitis, periarthritis, bursitis, contractures and muscular pains, articular pains and blocks, delays in consolidation of bony fractures, bony calluses et cetera a new therapy has been experimented with for years with shock waves of the focal type, being transmitted to the patient's body through acoustic reflectors reflecting and concentrating acoustic waves on a very small area of the skin surface facing the affected tissues, or with shock-waves of the radial type, being transmitted over an area of the skin surface overlooking the affected tissues through mechanical percussion made with a certain frequency by means of a solid and generally metallic body.

Examples of instruments for radial shock wave treatment are described in documents DE 10215416 A, US 5727556 A, and US 6413230 B 1.

The instrument described in DE 10215416 A comprises a beater part which generates extracorporeal pressure waves striking against a transmission element preferably made of steel for injecting the pressure waves into the patient's body. The transmission element has a part with a concave surface whose contour is put into direct contact with the patient's body. Said concave surface can be free or covered by a gel or by a dough in order to avoid air inclusions and to obtain a flat surface.

The instrument described in US 5727556 A includes a pulse-like wave generator. According to a first embodiment the pressure pulse source has an approximately pot-shaped housing filled with water; a planar membrane containing an electrically conductive material and lying against an electrical coil is immersed inside the water and emanates planar shock waves; a stationary lens present in the water converges the shock waves on a focus zone. According to a second embodiment a ram driven by an electromagnet supplied by current pulses strikes the patient's body applying pressure pulse to the same; the ram can strike the patient's body directly or through a coupling member such as a disk composed of a hydrogel. According to a third embodiment spherical projectiles formed e.g. of cork, cotton wedding, hollow plastic ball, are thrown against the patient's body. According to another embodiment a piston compresses an air volume causing a pulse-like air stream to emerge through a nozzle and to strike the patient's body. According to a further embodiment a piston compresses a liquid volume causing a pulse-like sprinklings to emerge through a duct of a barrel and to strike the patient body.

The instrument described in US 6413230 B1 includes a beater part capable of translations in a cylindrical chamber, arranged for striking against a transmission element comprising a metal probe with a blunt probe tip having an exit boundary surface which induces non-focused pressure waves into the patient's body, said beater part displacing said probe tip by less than 1 mm.

Radial shock wave treatment, the most widely used, has proved to be very effective. However, it frequently involves bothersome and sometimes compromising side effects such as the onset of painful sensations and hematomas in the part of the patient's body subjected to therapy.

The instrument disclosed in the preamble of claim 1 is known from the document WO 01/00094.

The general purpose of the present invention is to remedy the above-mentioned shortcomings by making available an instrument for the medical treatment of biological tissues by means of shock-wave therapy which is virtually free of the above-mentioned drawbacks as well as considerably less costly compared with similar instruments seen on the market.

The instrument in accordance with the present invention for medical treatment of biological tissues by means of shock-wave therapy, is of the type including a membrane (2) to put into contact with the part of the patient's body to be subjected to therapy for inputing therein the shock waves, and a device for production of said shock waves, said membrane being placed so as to close one of the two bases of a cylindrical chamber, and said device comprising:
- a piston (3) capable of translation in the above-mentioned cylindrical chamber and whose head is turned towards the above-mentioned membrane (2),
- a biocompatible fluid occupying the space (4) of the cylindrical chamber included between the piston head and the membrane (2), and
- a striking body (5) designed to strike the tail of the piston (3) with predetermined frequency.

The instrument is characterized in that:
- the above-mentioned membrane (2) is elastic and protrudes outside said one of the two bases of the cylindrical chamber so as to assume a convex form, in order to provide a good adaptability to the parts of the patient's body to be treated,
- said piston can move towords said membrane without elastic constraints present in the cylindrical chamber.

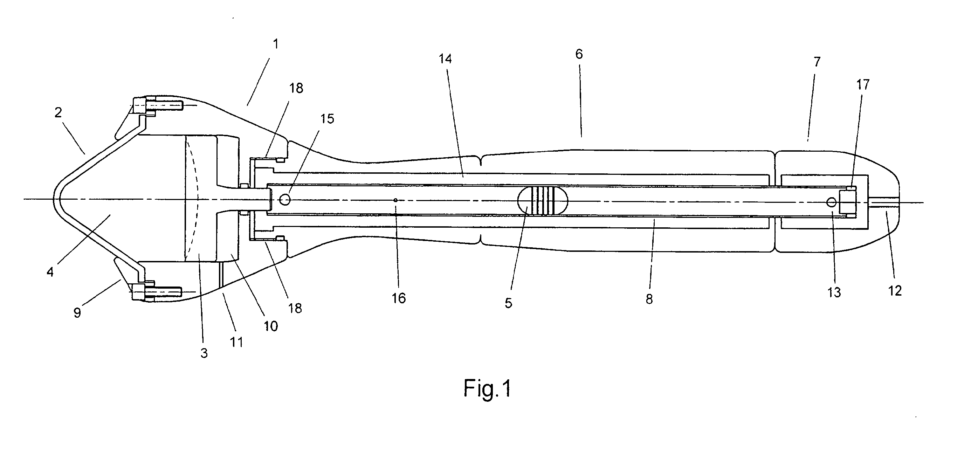

The characteristics of the present invention will be clarified by the following description of a non-limiting preferred embodiment given with reference to the annexed drawing (FIG 1) showing a cross-section view thereof.

The main components of this example are the following.
1. Head of the instrument in which a cylindrical chamber has its seat,
2. elastic membrane for example of latex or other rubber placed to close said cylindrical chamber at a base thereof and designed to be placed in contact with the patient's body part to be subjected to therapy,
3. piston capable of translation in the cylindrical chamber and whose head is turned towards the elastic membrane 2,
4. space of the cylindrical chamber included between the piston head 3 and the elastic membrane 2 and occupied by biocompatible fluid consisting for example of Vaseline, and
5. percussion body (shown in the figure in the form of a projectile) designed to strike the tail of the piston 3 with a predetermined frequency.
   The other marked parts of FIG 1 are the following.
6. Intermediate part of the instrument acting as the handle,
7. tail part of the instrument,
8. tube acting as the guide of the striker body 5 and housed in two cylindrical spaces in the parts 6 and 7,
9. ringnut for fastening the membrane 2,
10. space in the head 1 behind the tail side of the piston 3 in which there is air,
11. air breather hole in the space 10,
12. duct for inlet into the instrument of compressed air fed through a flexible tube not shown in the figure,
13. compressed air inlet hole in the right end of the tube 8,
14. chamber acting as a compressed air accumulation tank,
15. hole in the left end of the tube 8 which puts the space inside the tube in communication with the chamber 14,
16. hole allowing inlet of compressed air to the chamber 14, and
17. elastic body (for example rubber) acting as end-of-travel stop of the striker 5.

When the instrument is not active, supply of compressed air into the duct 12 is stopped by a shutoff valve in the compressed-air delivery apparatus and fitted upstream of the flexible supply tube of the duct 12. The shutoff valve and the delivery apparatus are not shown in the figure.

When the instrument is started, the shutoff valve starts a series of switchings from the closed condition to the open condition with a frequency selectable in a range from approximately 0.5 Hz to 5 Hz and equal to the percussion frequencyof the striker 5 on the piston 3 and with an open time lasting a fraction of a second and less than the time used by the striker to complete its forward movement against the tail of the piston 3.

At the moment of opening of the shutoff valve, the compressed air (whose pressure is selectable in a range of 1 bar to 5 bar approximately) enters through the duct 12 and the hole 13 in the tube 8 to cause projection of the striker 5 against the tail of the piston 3. When during its travel the striker passes beyond the hole 16, compressed air is inlet to the chamber 14 through the same hole.

Once the striker has struck the tail of the piston 3, it rebounds sufficiently to not obstruct the opening of the hole 15. In this manner, the compressed air accumulated in the meantime in the chamber 14 and coming out of the hole 15 returns the striker 5 to its starting position exactly against the end-of-travel stop 17 so as to be ready to begin the next percussion cycle.

In the figure the broken line in the piston 3 indicates a concave surface of its head. But this particular shape is not binding. On the contrary, a different curvature or a different form - for example flat or concave - can be assigned to influence the characteristics of the shock waves generated and in particular their focusing point that can be inside the membrane 2, or near its tip or outside the membrane itself.

As the head 1 of the instrument is assemblable onto or disassemblable from the handle 6 by simply screwing or unscrewing (the heavy lines 18 of FIG 1 indicate a threading on the opposing surfaces of the head and handle) the instrument in accordance with the present invention can be equipped with several interchangeable heads having the piston head surface of different shape to make available various types of shock forms.

It is clear from the above description that the instrument in accordance with the present invention can offer the following advantages:
- good adaptability of the elastic membrane to the surface to be treated thanks also to the presence of the internal fluid,
- capability to achieve better penetration of the shock waves by increasing the pressure exerted by the operator on the instrument without thereby causing severe pains and the deep hematomas typical of similar prior art instruments, and
- capability to generate various types of shock wave thanks to the capability of the instrument to be equipped with interchangeable heads having pistons with heads of different shapes.

Naturally numerous modifications, adaptations, variants, omissions and replacements of members by others functionally equivalent can be made to the above-mentioned embodiment without abandoning the scope of the invention which is defined by the claims.

One of these variants could concern for example the elastic membrane which instead of a conical shape with rounded top as shown in the drawing could take on another shape, for example a spherical shape.

Another variant could concern the means of operating the striker which could be for example of the hydraulic or electromagnetic type instead of the compressed air type.

Another variant could involve the manner of assembly of the instrument head on the handle which instead of by screwing could be by joint.

## Claims

1. Instrument for the medical treatment of biological tissues by means of shock-wave therapy, including a membrane (2) to put into contact with the part of the patient's body to be subjected to therapy for inputing therein the shock waves, and a device for production of said shock waves, said membrane being placed so as to close one of the two bases of a cylindrical chamber, and said device comprising:
- a piston (3) capable of translation in the above-mentioned cylindrical chamber and whose head is turned towards the above-mentioned membrane (2),
- a biocompatible fluid occupying the space (4) of the cylindrical chamber included between the piston head and the membrane (2), and
- a striking body (5) designed to strike the tail of the piston (3) with predetermined frequency,
**characterized in that**:
- the above-mentioned membrane (2) is elastic and protrudes outside said one of the two bases of the cylindrical chamber so as to assume a convex form, in order to provide a good adaptability to the parts of the patient's body to be treated.

2. Instrument for the medical treatment of biological tissues in accordance with claim 1 **characterized in that** its head is assemblable manually with the remaining part of the instrument by screwing or joint and by the fact that it is equipped with multiple interchangeable heads having pistons with different head forms and in particular concave or flat or convex in order to be able to generate shock waves with different characteristics and in particular with focal point in the elastic membrane (2) or near its tip or outside thereof.

3. Instrument for the medical treatment of biological tissues in accordance with claim 1 **characterized in that** the above-mentioned biocompatible fluid consists of Vaseline.

4. Instrument for the medical treatment of biological tissues in accordance with claim 1 **characterized in that** the above-mentioned membrane (2) is of latex.

5. Instrument for the medical treatment of biological tissues in accordance with claim 1 **characterized in that** the above-mentioned predetermined frequency with which the striker body (5) strikes the tail of the piston (3) can be selected between 0.5Hz and 5Hz.

## Patentansprüche

1. Instrument zur medizinischen Behandlung biologischen Gewebes mittels Schockwellentherapie, umfassend eine Membran (2), die mit dem Teil des Körpers eines Patienten in Kontakt zu bringen ist, welcher der Therapie unterzogen werden soll, um darin Schockwellen einzugeben, und eine Vorrichtung zur Erzeugung der Schockwellen, wobei die Membran angeordnet ist, um eine der beiden Grundflächen einer zylindrischen Kammer zu schließen, und die Vorrichtung folgendes umfasst:
- einen Kolben (3), der sich in der oben genannten zylindrischen Kammer verschieben kann und dessen Kopf der oben genannten Membran (2) zugewandt ist;
- ein körperverträgliches Fluid, die den Raum (4) der zylindrischen Kammer einnimmt, der zwischen dem Kolbenkopf und der Membran (2) eingeschlossen ist; und
- einen Schlagkörper (5), der dazu gedacht ist, mit einer vorbestimmten Frequenz an das Endstück des Kolbens (3) anzuschlagen;
**dadurch gekennzeichnet, dass**:
- die oben genannte Membran (2) elastisch ist und außerhalb einer der beiden Grundflächen der zylindrischen Kammer hervorragt, um eine konvexe Form anzunehmen, um eine gute Anpassungsfähigkeit an die zu behandelnden Teile des Körpers eines Patienten bereitzustellen.

2. Instrument zur medizinischen Behandlung biologischen Gewebes nach Anspruch 1, **dadurch gekennzeichnet, dass** sein Kopf mit dem restlichen Teil des Instruments durch Verschrauben oder Aneinanderfügen manuell zusammengebaut werden kann und dass es mit mehreren austauschbaren Köpfen ausgestattet ist, die Kolben mit unterschiedlichen, und insbesondere konkaven oder flachen oder konvexen Kopfformen aufweisen, um Schockwellen mit unterschiedlichen Merkmalen, und insbesondere mit einem Fokus in der elastischen Membran (2) oder in der Nähe ihrer Spitze oder außerhalb davon, zu erzeugen.

3. Instrument zur medizinischen Behandlung biologischen Gewebes nach Anspruch 1, **dadurch gekennzeichnet, dass** das oben genannte körperverträgliche Fluid aus Vaseline besteht.

4. Instrument zur medizinischen Behandlung biologischen Gewebes nach Anspruch 1, **dadurch gekennzeichnet, dass** die oben genannte Membran (2) aus Latex besteht.

5. Instrument zur medizinischen Behandlung biologischen Gewebes nach Anspruch 1, **dadurch gekennzeichnet, dass** die oben genannte vorbestimmte Frequenz, mit der der Schlagkörper (5) an das Endstück des Kolbens (3) schlägt zwischen 0,5 Hz und 5 Hz wählbar ist.

## Revendications

1. Instrument de traitement médical de tissus biologiques par une thérapie à ondes de choc, incluant une membrane (2) à mettre en contact avec la partie du corps du patient à soumettre à la thérapie pour y introduire les ondes de choc, et un dispositif de production des ondes de choc, la membrane étant placée de façon à fermer l'une des deux bases d'une chambre cylindrique, et le dispositif comprenant :
un piston (3) apte à se déplacer en translation dans la chambre cylindrique susmentionnée dont la tête est tournée vers la membrane (2) susmentionnée ;
un fluide biocompatible occupant l'espace (4) de la chambre cylindrique inclus entre la tête de piston et la membrane (2) ; et
un corps de frappe (5) destiné à frapper la queue du piston (3) à une fréquence prédéterminée ;
**caractérisé en ce que** la membrane susmentionnée (2) est élastique et fait saillie à l'extérieur de l'une des deux bases de la chambre cylindrique de façon à prendre une forme convexe pour fournir une bonne adaptabilité aux parties du corps du patient à traiter.

2. Instrument de traitement médical de tissus biologiques selon la revendication 1, **caractérisé en ce que** sa tête peut être assemblée manuellement avec la partie restante de l'instrument par vissage ou jonction et **en ce qu'**il est équipé de têtes multiples interchangeables ayant des pistons de formes de tête différentes et en particulier concaves, plats ou convexes pour permettre de produire des ondes de choc de caractéristiques différentes et en particulier avec un point focal dans la membrane élastique (2) ou près de son extrémité ou à l'extérieur de celle-ci.

3. Instrument de traitement médical de tissus biologiques selon la revendication 1, **caractérisé en ce que** le fluide biocompatible est de la vaseline.

4. Instrument de traitement médical de tissus biologiques selon la revendication 1, **caractérisé en ce que** la membrane (2) susmentionnée est en latex.

5. Instrument de traitement médical de tissus biologiques selon la revendication 1, **caractérisé en ce que** la fréquence prédéterminée susmentionnée à laquelle le corps de frappe (5) frappe la queue du piston (3) peut être choisi entre 0,5 Hz et 5 Hz.
